# EUROPEAN PATENT SPECIFICATION

(11) **EP 4 247 244 B1**
(45) Date of publication and mention of the grant of the patent: **19.11.2025**
(21) Application number: 21895889.0
(22) Date of filing: 19.11.2021
(51) Int. Cl.: A61L 15/42, A61L 15/58, A61B 5/01, A61B 5/257, A61B 5/145, A61B 5/024, A61B 5/11, A61B 5/021, A61B 5/1455

(54) **WEARABLE PATCHES AND DEVICES**
TRAGBARE PFLASTER UND VORRICHTUNGEN
PATCHS ET DISPOSITIFS POUVANT ÊTRE PORTÉS

(30) Priority: 23.11.2020 US 202063117112 P
(43) Date of publication of application: 27.09.2023
(73) Proprietor: Henkel AG & Co. KGaA, 40589 Düsseldorf (DE)
(72) Inventor: KREJSA, Michael, Bridgewater, New Jersey 08807 (US); PALLIARDI, Mary E., Rocky Hill, Connecticut 06067 (US); MESSANA, Andrew D., Rocky Hill, Connecticut 06067 (US); LAYSER, Kathryn, Bridgewater Township, New Jersey 08807 (US)
(86) International application number: PCT/US2021/072505
(87) International publication number: WO 2022/109596

(56) References cited:
- WO-A1-2016/019250
- US-A1- 2005 169 975
- US-A1- 2009 076 363
- US-A1- 2014 005 617
- US-B1- 6 262 330
- US-B1- 6 558 790

## Description

### FIELD OF THE INVENTION

The invention relates to extended wear skin patches. Health monitoring detectors can further be adhered onto the patches to monitor the health of the wearer.

### BACKGROUND OF THE INVENTION

Wearable devices provide information to the wearer, or in some cases transmit this information to a secondary device. These devices can be directed to medical, personal healthcare, fitness/wellness, and sports activities, always targeting the human body monitoring, with minimal discomfort and interference with normal human activities.

This information can be used to monitor, improve or warm the wearer's state of health and may involve interaction with a clinician, physician, personal trainer or other health and/or wellness professionals. These devices can be directed to applications including medical, personal healthcare, fitness/wellness, and sports activities, and involve monitoring monitor of human health characteristics (such as but not limited to blood sugar level, pulse rate, and heart rate) with minimal discomfort and interference with normal human activities.

It is preferable that these wearable devices are non-invasive and easy to use and to collect information. In fact, such devices that allow or continuous or extended (days/weeks) monitoring is desirable. The devices create a synergy between multiple science domains such as biomedical technologies, micro and nanotechnologies, materials engineering, electronic engineering and information and communication technology.

A device is formed by attaching a detector onto a wearable patch, which is in direct contact with the wearer. In some aspects, the patch is adhered onto the dermis of the wearer with an adhesive matrix. The patch with the adhesive matrix must feature good conformability to skin for a prolonged time with low skin irritation during and removal from the skin. Long term bonding to the dermis can result in adhesive related skin damage, a phenomenon known as MARSI (Medical adhesive Related Skin Injury).

U.S. Patent 6,262,330 B1, describes a long wear construction having a water-vapor transmission rate of at least 500 (g/m^2)/day from the adhesive to provide long wear times. It is generally understood in the art that long wear applications require high moisture value transmission rate (MVTR).

Accordingly, there is a need in the art for extended time, wearable patches that minimizes MARSI. The current invention fulfills this need.

### BRIEF SUMMARY OF THE INVENTION

The invention provides extended wear skin patches and devices comprising extended wear skin patches. The extended wear skin patches and devices include a facestock and an adhesive matrix that may be attached onto the dermis of the wearer.

One aspect of the invention is directed to an extended wear skin patch prepared with:
(A) a flexible nonwoven facestock and
(B) a hybrid adhesive matrix having acrylic polymers copolymerized with rubber polymers, wherein the hybrid adhesive matrix has a moisture vapor transmission rate less than 400 (g/m²)/day, wherein the moisture vapor transmission rate is measured with an inverted cup method with adhesive coating on 50 g/m² as described herein.
The extended wear skin patch has a wear time greater than 14 days at adhesion level greater than 4 according to a wear rating scale.

In another aspect of the invention is directed to a device comprising:
a. an extended wear skin patch comprising
   i. an adhesive matrix having a moisture vapor transmission rate less than 400 (g/m²)/day, wherein the moisture vapor transmission rate is measured with an inverted cup method with adhesive coating on 50 g/m² as described herein; and
   ii. a flexible polyurethane nonwoven facestock;
b. a second adhesive; and
c. a detector.
The first adhesive matrix with direct skin contact has a moisture vapor transmission rate less than 400 (g/m²)/day. The second adhesive adheres the detector to the nonwoven facestock.

Another aspect of the invention is directed to a method of forming a patch comprising:
(A) preparing a polyurethane non-woven facestock having a first side and a second side;
(B) preparing a release liner having a first side and a second side, wherein a silicone release coating is applied onto the first side;
(C) preparing a first adhesive having a moisture vapor transmission rate less than 400 (g/m²)/day, wherein the moisture vapor transmission rate is measured with an inverted cup method with adhesive coating on 50 g/m² as described herein;
(D) coating the first adhesive onto the release coating on the first side of the release liner; and
(E) laminating the first adhesive onto the first side of the polyurethane non-woven facestock.
whereby the adhesive is sandwiched between the first side of the polyurethane non-woven facestock and the release coating on the first side of the release liner.
A detector may further be adhered onto the second side of the polyurethane non-woven facestock with a second adhesive.

### BRIEF DESCRIPTION OF THE DRAWINGS

Figure 1 is a schematic drawing of a wearable patch.
Figure 2 is a photograph of a device adhered onto a wearer.

### DETAILED DESCRIPTION OF THE INVENTION

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present disclosure. The materials, methods, and examples disclosed herein are illustrative only and not intended to be limiting.

As used in the specification and in the claims, the term "comprising" may include the embodiments "consisting of and "consisting essentially of." The terms "comprise(s)," "include(s)," "having," "has," "can," "contain(s)," and variants thereof, as used herein, are intended to be open-ended transitional phrases, terms, or words that require the presence of the named ingredients/steps and permit the presence of other ingredients/steps. However, such description should be construed as also describing compositions or processes as "consisting of" and "consisting essentially of" the enumerated ingredients/steps, which allows the presence of only the named ingredients/steps, along with any impurities that might result therefrom, and excludes other ingredients/steps.

Numerical values in the specification and claims of this application, particularly as they relate to polymers or polymer compositions, reflect average values for a composition that may contain individual polymers of different characteristics. Furthermore, unless indicated to the contrary, the numerical values should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 to 10" is inclusive of the endpoints, 2 and 10, and all the intermediate values). The endpoints of the ranges and any values disclosed herein are not limited to the precise range or value; they are sufficiently imprecise to include values approximating these ranges and/or values. As used herein, approximating language may be applied to modify any quantitative representation that may vary without resulting in a change in the basic function to which it is related. Accordingly, a value modified by a term or terms, such as "about," may not be limited to the precise value specified, in some cases. In at least some instances, the approximating language may correspond to the precision of an instrument for measuring the value. The modifier "about" should also be considered as disclosing the range defined by the absolute values of the two endpoints. For example, the expression "from about 2 to about 4" also discloses the range "from 2 to 4." The term "about" may refer to plus or minus 10% of the indicated number. For example, "about 10%" may indicate a range of 9% to 11 ", and "about 1" may mean from 0.9-1.1. Other meanings of "about" may be apparent from the context, such as rounding off, so, for example "about 1" may also mean from 0.5 to 1.4.

As used herein, the term "pressure-sensitive adhesive" or "PSA" refers to a viscoelastic material which adheres instantaneously to most substrates with application of slight pressure and remains permanently tacky. A polymer is a pressure sensitive adhesive within the meaning of the term as used herein if it has the properties of a pressure sensitive adhesive per se or functions as a pressure sensitive adhesive by admixture with other components.

Weight percent, "wt%," indicates the weight percent based on the total weight of the components in an adhesive.

As used herein, "health characteristic" is defined as some measurable physiological sign that is related to health, fitness or wellness. This can include, but is not limited to, blood pressure, pulse, heart rate, dissolved oxygen content and blood sugar levels.

Extended wear time is defined as remaining adhered on the dermis for two-weeks or more as measured in clinical wear trials conditions.

Clinical trial evaluations of the patches are conducted with 1" by 3" test strip with rounded corners (no hard corners since they are prone to be picked off the surface). Typically, a total of six to eight patches are tested per clinical trial. The patches are applied on the inside lower (volar) forearms, and applied on any of the numbered locations, shown below.

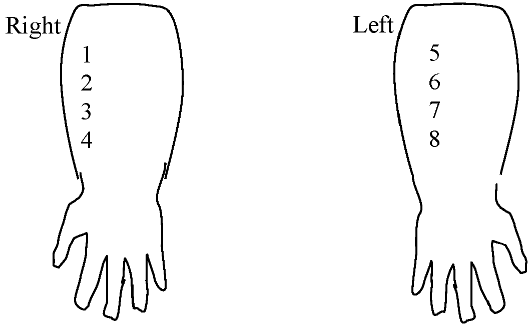

The number of individuals included in the clinical trial must be a minimum of five (5) individuals, preferably ten (10) or more individuals, and most preferably fifteen (15) or more individuals.

The test area is first cleaned, e.g., with an isopropanol or similar wipe which is allowed to dry for a minimum of 60 seconds, before a patch is applied. The patch is applied by separating the release liner from the adhesive at one end, applying said end to the inside/center of the body side of the arm, and then pulling the patch towards the outside of the arm by pulling the release liner. This "lays down" the patch in a clean manner. Finger pressure is then used along the entire patch to ensure a good initial lay down/bonding to the skin surface.

No creams or lotions are to be used or applied to the test area within 24 hours of the patch application nor during the testing.

The individuals in the clinical trial are permitted to conduct normal activities including exercise. No aggressive cleaning or washing of this area is permitted but light dabbing is permitted.

Evaluations of the patch adherence onto the arm are conducted throughout the clinical trial period. For a three week wear clinical trial, evaluations of wear conditions should be conducted every three to four days using a wear rating scale.

Wear Rating Scale are recorded at each evaluation time using the scale shown below.

| **Grade** | **Adhesion** |
|---|---|
| 0 | Dressing off |
| 1 | Dressing almost (hanging) off |
| 2 | ¾ of dressing off |
| 3 | ½ of dressing off |
| 4 | ¼ of dressing off |
| 5 | 3 to 4 edges lifted |
| 6 | 1 to 2 edges lifted |
| 7 | All corners adhering firmly |

It is typically understood in the art that high MVTR values in adhesives lend to extended wear time. Acrylic-based adhesives provide high MVTR values. Rubber based technologies, such as solvent rubber and rubber based hot melts, exhibit both low MVTR values and are generally known to provide short wear times on skin. While manipulating the coating patterns of the adhesives, e.g., non-continuous adhesive coating, on a substrate can increase MVTR values, they do not provide significant increase in skin wear time.

One aspect of the invention is directed to an extended wear skin patch. The extended wear skin patch includes a facestock and a pressure sensitive adhesive matrix that may be attached onto the dermis of the wearer. The wearer may be human, animal, including pet, livestock, zoo animal, wild animal; plant, and the like.

In one embodiment, the pressure sensitive adhesive matrix is a formed from an acrylic-based adhesive. The acrylic and methacrylic components are limited only by the requirement that the polymer composition must result in a polymer with pressure sensitive adhesive properties. Common acrylic and methacrylic monomers include, but are not limited to, methyl acrylate, methyl methacrylate, ethyl acrylate, ethyl methacrylate, isopropyl acrylate, butyl acrylate, butyl methacrylate, hexyl acrylate and 2-ethylhexyl acrylate. The acrylic polymer may also contain a functional monomer for the purpose of improving adhesion and also potentially for crosslinking. Other functional monomers for adhesion promotion may also be included. One such example includes, but is not limited to, tertiary amine functionalized alkyl acrylate monomers.

In another embodiment, the pressure sensitive adhesive matrix is a formed from a solvent-based acrylic-based adhesive. The acrylic and methacrylic components mentioned earlier may be formed in solvent, and later removed to form the pressure sensitive adhesive. Common solvents include ethyl acetate, acetone, hexane, cyclohexane, heptane, toluene, ethanol, and isopropyl alcohol, or a combination thereof.

In yet another embodiment, the pressure sensitive adhesive matrix is formed from an acrylic-rubber polymer. The term "acrylic-rubber polymer" can thus refer to any combination of acrylic and methacrylic monomers covalently bonded to the rubber component. The acrylic-rubber hybrid is prepared using standard acrylic (and methacrylic) monomers comprising acrylates and methacrylate monomers, having side chain lengths of from 1 to 20 carbons, more commonly from 1 to 8 carbons; and a rubber component comprising a hydrogenated olefin rubber. For the acrylic and methacrylic components in the acrylic-rubber hybrid polymer, common monomers include, but are not limited to, methyl acrylate, methyl methacrylate, ethyl acrylate, ethyl methacrylate, isopropyl acrylate, butyl acrylate, butyl methacrylate, hexyl acrylate and 2-ethylhexyl acrylate. They may also contain a functional acrylic and methacrylic monomer for the purpose of improving adhesion, and also potentially for crosslinking. Other functional monomers for adhesion promotion may also be included. Typical functional monomers for this acrylic and methacrylic portions of the acrylic-rubber hybrid polymer include, but are not limited to, acrylic acid, methacrylic acid, beta-carboxyethyl acrylate, 2-hydroxyethyl acrylate, 2-hydroxyethyl methacrylate, hydroxypropyl acrylate, hydroxybutyl acrylate, and tertiary amine functionalized alkyl acrylate monomers. The acrylic component of the acrylic-rubber polymer may also include other acrylic monomers capable of copolymerizing with the above monomers. These can include, but are not limited to, styrene, vinyl acetate and N-vinyl pyrrolidone. The rubber component of the acrylic-rubber hybrid polymer may be based on any combination of isoprene, butadiene, isobutylene, hydrogenated isoprene, hydrogenated butadiene or hydrogenated isobutylene monomers. The rubber component is not limited by the isomer distribution of the olefin monomers; as an example, butadiene may be present as either the hydrogenated version of the 1,2 isomer or as the hydrogenated versions of the cis-1,4 and trans-1,4 isomers.

The acrylic, solvent-based acrylic, and acrylic-rubber polymer may each further be combined with plasticizers and tackifiers to improve adhesive characteristics of the pressure sensitive adhesive.

Examples of plasticizers include fatty acid esters of monohydric alcohols, such as cetyl octanoate, hexyl laurate, isopropyl myristate, isopropyl palmitate, butyl stearate and myristyl lactate; dibasic acid esters such as dioctyl adipate, diethyl sebacate, dioctyl sebacate and dioctyl succinate; and fatty acid esters of polyhydric alcohols such as propyleneglycol dicaproate, glyceryl trioctanoate, glyceryl tri(octanoate/decanoate), medium chain fatty acid triglycerides and the like, among which fatty acid esters such as isopropyl myristate, isopropyl palmitate.

Suitable tackifying agents include: (1) aliphatic hydrocarbons; (2) mixed aliphatic and aromatic hydrocarbons; (3) aromatic hydrocarbons; (4) substituted aromatic hydrocarbons; (5) hydrogenated esters; (6) polyterpenes; and (7) wood resins or rosins and hydrogenated forms thereof.

Those skilled in the art will appreciate that each polymer will include minor amounts of polymerization initiators that are used in preparing or stabilizing the random copolymers of the disclosure. Polymerization initiators that are suitable for producing random polymerization are known in the art. Particularly preferred polymerization initiators include 2,2'-azodi(2-methylbutyronitril) (AMBN), dibenzoyl peroxide, lauryol peroxide, and 2,2'-azobisisobutyronitrile (AIBN).

The polymerization process can be optionally carried out in the presence of a solvent. Also, the processes can be carried out "neat," that is, not in the presence of any solvent. In other embodiments, the processes can be carried out in the presence of a solvent. Preferred solvents are organic solvents, for example, ethyl acetate, acetone, hexane, cyclohexane, heptane, toluene, ethanol, and isopropyl alcohol, or a combination thereof.

An antioxidant or stabilizer may also be included in the compositions described herein in amounts of up to about 3% by weight, more typically in amounts of about 0.5%. Among the stabilizers or antioxidants useful herein are the hindered phenols or hindered phenols in combination with a secondary antioxidant such as distearyl thiodipropionate ("DSTDP") or dilauryl thio-dipropionate ("DLTDP"). Representative antioxidants include: 1,3,5-trimethyl 2,4,6-tris (3,5-di-tert-butyl-4-hydroxybenzyl)benzene; pentaerythrityl tetrakis-3(3,5-di-tert-butyl-4-hydroxyphenyl)propionate; pentaerythritol tetrakis (3-lauryl thiodipropionate); n-octadecyl-3,5-di-tert-butyl-4-hydroxyphenol)-propionate; 4,4'-methylenebis (2,6-tert-butylphenol); 4,4'-thiobis (6-tert-butyl-o-cresol); 2,6-di-tertbutylphenol; 6-(4-hydroxyphenoxy)-2,4-bis(n-octyl-thio)-1,3,5-triazine; di-n-octadecyl 3,5-di-tert-butyl-4-hydroxy-benzyl-phosphonate; 2-(n-octylthio)ethyl 3,5-di-tert-butyl-4-hydroxy-benzoate; and sorbitol hexa[3-(3,5-di-tert-butyl-4-hydroxyphenyl)-propionate]. Preferred are IRGAFOS 168, a secondary antioxidant available from Ciba and IRGANOX 1010, a hindered phenol primary antioxidant available from Ciba-Geigy. Other antioxidants include ETHANOX 330, a hindered phenol from Albermarle; 2,5 di-tert-amyl hydroquinone; and NAUAGARD P a tris (p-nonylphenyl)phosphite from Adivant. Preferred antioxidants include butylated hydroxyanisole (BHA) and butylated hydroxytoluene (BHT).

These pressure sensitive adhesive compositions can optionally include at least one ingredient selected from the group consisting of enhancers, viscosity modifiers, excipients, diluents, emollients, anti-irritants, opacifiers, and pigments, which may be incorporated in minor or larger amounts into the adhesive formulation, depending on the purpose.

The pressure sensitive adhesive is bonded onto the first side of a facestock to form a patch. The facestock is a flexible substrate. The flexible substrate of the patch is used as the support layer. This can be made from polyethylene, polypropylene, polyethylene terephthalate (hereinafter, 'PET' may also be referred to.), polybutylene terephthalate, polyethylene naphthalate, polystyrene, nylon, cotton, rayon acetate, rayon, rayon/polyethylene terephthalate composite, polyacrylonitrile, polyvinyl alcohol, acrylic polyurethane, polyester-based polyurethane, polyether polyurethane, styrene - isoprene - styrene copolymer, styrene - butadiene - styrene copolymer, styrene - ethylene - propylene - styrene copolymer, styrene - butadiene rubber, ethylene - vinyl acetate copolymer, of film-like cellophane film materials may be used in single or multiple layers. Flexible substrates, and particularly, laminated fabric are preferably used as facestock. Fabrics, other natural fibers, polyester fibers, polyacrylic fibers, polyurethane fibers non-woven fabric, woven fabric and knitted fabric and, among the elastic polyurethane fiber which has a non-woven fabric is preferable. Laminated fabric, a film laminated with the fabric, may also be the basis of the facestock.

Polyethylene terephthalate (PET) based non-wovens are the standard non-woven materials used as medical facestock for device, wearables and monitoring applications involving skin contact. They are highly breathable materials that provide good load bearing capabilities. For medical device, wearable and medical monitoring patches, medical substrates capable of bearing the device or patch load are used. Constructions with PET based non-wovens show good wear characteristics, but even with optimized tape constructions are typically limited to less than two-week standard wear characteristics.

Other non-woven technologies are possible and available for skin contact medical applications including, but not limited to, polyurethane-based non-wovens. Materials used for these medical tape applications must be suitable for skin contact. While many different flexible substrates can be used, polyurethane-based facestock is preferred. The polyurethane facestock is made from a non-woven fibers produced using polyurethane chemistries. The non-woven material used in this invention is commercially available material from Freudenberg Performance Materials GmBH and are sold under the M1600 series product names.

The thickness of the facestock ranges from about 0.15 to about 0.25 mm (150 µm to 250 µm) with a weight of 50 to 125 grams/m^2. For a laminate comprising the film and the fabric, the thickness may be 200-500µm, and more preferably 250-400 µm, If the thickness is too thin, the base material is too flexible, resulting in a pressure sensitive adhesive tape for skin which is unable to maintain the detector load. If the thickness is too great, the flexibility of the base material is impaired, resulting in a pressure sensitive adhesive tape for skin deteriorated in conformability to skin and which exhibits shorter wear due to the ability to confirm appropriately to skin movement

It is generally regarded that long wear medical adhesives should have high breathability. This can be measured and defined as Moisture Vapor Transmission Rate (MVTR). Higher MVTR rates indicate higher breathability. MVTR values depend on the adhesive, adhesive thickness, and the test method. Standard adhesives have MVTR values in the 300 to 900 (grams/square meter)/day {(g/m^2)/day}, with high MVTR rates being defined as over 900 (g/m^2)/day, and over 1200 g/m^2)/day as very high. Low MVTR adhesives are defined as adhesives having an MVTR under 300 (g/m^2)/day, and typically are below 100 (g/m^2)/day. Adhesives with MVTR values below 100 (g/m^2)/day typically have a very short skin adhesion wear times, on the order of 24 hours or less. Surprisingly, the combination of a polyurethane non-woven facestock coated with an adhesive having MVTR values less than 400 (g/m^2)/day provide extended wear time. Given that this combination has low MVTR, specifically, less than 400 (g/m^2)/day, it is generally understood to have poor long wearability. However, this combination allows for adhesion on dermis for at least 14 days.

In one embodiment, a patch may be formed by
(a) preparing a polyurethane non-woven facestock having a first side and a second side;
(b) preparing a release liner having a first side and a second side;
(c) preparing a first adhesive having a moisture vapor transmission rate less than 400 (g/m^2)/day;
(d) coating the first adhesive onto the first side of the release liner, wherein the first side of the release liner is coated with a silicone release coating;
(e) laminating the first adhesive onto the first side of the polyurethane non-woven substrate.
As a result, the adhesive is sandwiched between the first side of the polyurethane non-woven facestock and the first side of the release liner. The release liner is typically a siliconized released paper or siliconized released PET film

In another embodiment, the adhesive may be coated directly on to the facestock. Depending on the type of the adhesive, solvent evaporation may be required. Alternatively, the adhesive may be transfer coated on a liner and then transferred onto the facestock. The pressure sensitive adhesive is coated from about 10 to about 150 g/m^2, preferably from about 25 to about 75 g/m^2. A siliconized released paper or siliconized released PET film is then applied on top of the adhesive for use later.

The patch may be produced in any desirable unit form and shape. A circular form is convenient as it contains no corners which might be easily detached from the skin. A rectangular form or square forms, with or without rounded edges may also be formed. In one embodiment, the patch is a strip of one inch by 7.6 cm (three inches) in length and width. The patch can also be in a roll-like form, and may be shaped to accommodate the detector to form a device.

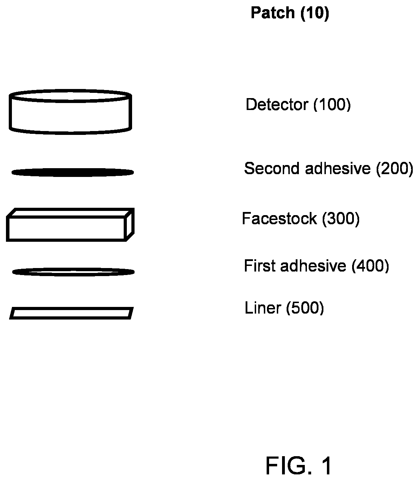

In another embodiment, a detector is further attached to the patch to form a device. As shown in Figure 1, a device (10) comprises a detector (100), which is attached onto the facestock (300) with a second adhesive (200). On the other side of facestock, first adhesive (400) layer is sandwiched with a liner (500). The liner (500) can be removed and the first adhesive can be adhered onto the dermis for extended wear. The first adhesive has a MVTR of less than 400 (g/m^2)/day.

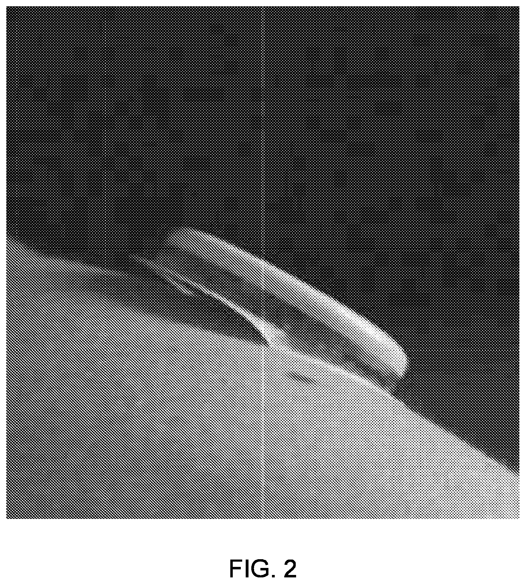

Figure 2 is a photograph of the device attached onto a wearer.

To form a medical patch including a detector, the patch undergoes additional steps of:
(f) preparing a second adhesive;
(g) preparing a second release liner, having a first surface and a second surface;
(h) coating the second adhesive onto the second surface of the second release liner;
(i) preparing a detector; and
(j) applying the detector onto the adhesive on the second surface of the second release liner.

The second adhesive may be any adhesive, ranging from a hot melt adhesive, solvent acrylic adhesive, solvent acrylic-rubber hybrid adhesive, emulsion acrylic adhesive or contact adhesive. Preferably the second adhesive complies with ISO 10093 requirements for biocompatibility on intact skin and has high adhesion to the detector. In some embodiment, the second adhesive may be the same as the first adhesive.

The detector on the device measures physiological signs, e.g., electrical signs, biochemical, and biosignals, to better understand the bodily health status and reaction to external factors. In another embodiment the detector may be an amplifier of signals. The detector attached to the substrate can be wearable in various scenarios: type of home/remote or clinical environment; type of monitoring (offline or online); and the type of user (healthy or patient). The detector may monitor heart rate, single-lead ECG blood pressure, heart rate variability, respiration rate, blood oxygen saturation, blood glucose, skin perspiration, capnography, body temperature, motion evaluation, cardiac implantable detectors, fall detection, activity, body posture, and ambient parameters. This information may be relayed directed to the wearer at the detector site or via transponder, or to a remote clinician. It is envisioned that the detector can monitor activity area for fitness/wellness and non-medical applications, including self-monitoring and rehabilitation procedures. The detector can provide prediction to better identify events that have not yet occurred yet to prevention problems, and sometimes or to support a diagnosis decision. The detector may also detect anomaly and raise alarms as soon as an anomaly is detected. The detector can provide clinical monitoring, resulting in a clinical decision according to retrieved knowledge of physiological signs, health records and anomaly detection data.

The specific embodiments described herein are offered by way of example only, and the invention is to be limited only by the terms of the appended claims, along with the full scope of equivalents to which such claims are entitled.

### EXAMPLES

Patches were constructed with various pressure sensitive adhesives, having different MVTR values, on different facestocks. Typical patch was constructed with a size of 1"x3", with rounded corners. The coating weight of the adhesive ranged from about 25 to 150 grams/square meter on the patch. A total of six to eight patches were made with various adhesives and facestock for each sample, listed in Table 1.

For the pressure sensitive adhesives, UV curable acrylic polymer based adhesive, acrylic rubber hybrid polymer based adhesive (LOCTITE DURO-TAK AH 115), solvent based acrylic polymer based adhesive, and rubber polymer-based adhesives were tested.

For facestock, polyurethane nonwovens (Freudenberg M1670), PET nonwovens, and polyurethane films were tested.

The MVTR values of the adhesives were measured with an Inverted Cup method with adhesive coating of 50 g/m^2 on a highly breathable flexible film facestock. For the MVTR measurements, a Payne Permeability Cup, Fisher Scientific, Catalog No. 13-338 was used. The coatings were conditioned at 22.2°C +/- 0.62°C [72°F (+/- 2°F)] and 50% relative humidity (+/- 5%) for a minimum of 16 hours prior to testing. The test involved filling a permeability cup with approximately 10 grams of water, applying the patch, adhesive side down, to the permeability cup, and weighing the cup initially and after 24 hours. The cup is maintained inverted for the 24 hours at 37.8 °C and 25% relative humidity. The measured MVTR are for each adhesive is listed in Table 2.

Patches were then tested in clinical trials. Six to eight patches for each sample in Table 1 were tested on the inside lower forearms, in a clinical study as described above. Evaluations of wearability were tested using the wear rating as shown in Table 1, and were evaluated every three days.

**Table 1: Wear Rating Scale**

| Grade | Adhesion |
|---|---|
| 0 | Dressing off |
| 1 | Dressing almost (hanging) off |
| 2 | ¾ of dressing off |
| 3 | ½ of dressing off |
| 4 | ¼ of dressing off |
| 5 | 3 to 4 edges lifted |
| 6 | 1 to 2 edges lifted |
| 7 | All corners adhering firmly |

For extended wear applications, the patch, as expected, showed some signs of edge lift, and some lift of the adhesive coated area, as tested in the clinical trials. A minimum average rating of at least grade 4 was acceptable, preferably greater than grade 5. Data reported in table 2 for wear time is the average wear time (in days) of all patches for a specific adhesive and facestock patch construction combination.

**Table 2**

| Patch | Adhesive (polymer) | Facestock | MVTR (g/m^2)/day | Wear Time (days) |
|---|---|---|---|---|
| A | UV hot melt acrylic | PET non-woven | 857 | 7 to 10 |
| B | UV hot melt acrylic | PET non-woven | 841 | 1 to 2 |
| C | Acrylic rubber hybrid (LOCTITE DURO-TAK AH 115) | PET non-woven | 97 | 12 to 13 |
| D | Acrylic rubber hybrid (LOCTITE DURO-TAK AH 115) | PU non-woven | 97 | 18 to 20 |
| E | Solvent acrylic | PET non-woven | 372 | 14 |
| F | Solvent acrylic | PU non-woven | 372 | 18 to 20 |
| G | Rubber (styrene-block copolymer) hot melt | PU Film | 194 | less than 1 |
| H | Rubber (hydrogenated styrene-block copolymer) hot melt | PU Film | 12 | 1 |

The MVTR value of the acrylic rubber hybrid adhesive (Patches C and D) was under 100 (g/m^2)/day. Based on the MVTR value above, such an adhesive would be expected to produce a very low wear adhesive; however, Patch D provided wear time of at least 18 days. Very high MVTR value adhesives, UV hot melt acrylic adhesives (Patches A and B) had lower wear times, despite their high MVTR values. Solvent acrylic polymer based adhesive, having a MVTR value of less than 400, applied onto polyurethane non-woven facestock also provided wear time of at least 18 days.

## Claims

1. An extended wear skin patch comprising:
(a) A polyurethane non-woven facestock; and
(b) hybrid adhesive matrix prepared with acrylic polymers copolymerized with rubber polymers, wherein the hybrid adhesive matrix has a moisture vapor transmission rate less than 400 (g/m²)/day
wherein the moisture vapor transmission rate is measured with an inverted cup method with adhesive coating on 50g/m² as described in the description.

2. The patch of claim 1, wherein the polyurethane non-woven substrate has a thickness of 150 to 250 µm and a weight of 50 to 125 g/m².

3. The patch of claim 1, wherein the acrylic polymer is prepared with a mixture of acrylate monomers selected from the group consisting of acrylic acid, methacrylic acid, beta-carboxyethyl acrylate, 2-hydroxyethyl acrylate, 2-hydroxyethyl methacrylate, hydroxypropyl acrylate, hydroxybutyl acrylate, tertiary amine functionalized alkyl acrylate monomers, and mixtures thereof; and wherein the rubber polymer is prepared from the group consisting of isoprene, butadiene, isobutylene, hydrogenated isoprene, hydrogenated butadiene, hydrogenated isobutylene, and mixtures thereof.

4. The patch of claim 3, wherein the adhesive further comprises plasticizer, tackifier, adhesion promotor, initiator, solvent, antioxidant, and additive.

5. The patch of claim 1, further comprising a release liner selected from the group consisting of siliconized released paper or siliconized released PET film.

6. The patch of claim 1, which is a medical patch.

7. A device comprising:
a. an extended wear skin patch comprising
i. an adhesive matrix having a moisture vapor transmission rate less than 400 (g/m²)/day, wherein the moisture vapor transmission rate is measured with an inverted cup method with adhesive coating on 50g/m² as described in the description; and
ii. a flexible polyurethane nonwoven facestock;
b. a second adhesive; and
c. a detector.

8. The device of claim 7, wherein the adhesive matrix is an acrylic-rubber hybrid adhesive or a solvent acrylic adhesive.

9. The device of claim 8, wherein the detector is suitable for detecting or measuring a physiological sign selected from the group consisting of an electrical change, biochemical change, temperature change, motion change, activity change, posture change, and mixtures thereof.

10. A method of forming an extended wear skin patch comprising:
a. preparing a polyurethane non-woven facestock having a first side and a second side;
b. preparing a release liner having a first side and a second side, wherein a silicone release coating is applied onto the first side;
c. preparing a first adhesive having a moisture vapor transmission rate less than 400 (g/m²)/day, wherein the moisture vapor transmission rate is measured with an inverted cup method with adhesive coating on 50g/m² as described in the description;
d. coating the first adhesive onto the release coating on the first side of the release liner; and
e. laminating the first adhesive onto the first side of the polyurethane non-woven substrate;
whereby the adhesive is sandwiched between the first side of the polyurethane non-woven facestock and the first side of the release liner.

11. The method of forming a patch of claim 10, wherein the first adhesive is prepared with an acrylic-rubber hybrid polymer, plasticizer, tackifier, adhesion promotor, initiator, and additive.

12. The method of forming a patch of claim 11, further comprising the steps after step d,
e. preparing a second adhesive;
f. preparing a second release liner, having a first surface and a second surface;
g. coating the second adhesive onto the second surface of the second release liner;
h. preparing a detector; and
i. applying the detector onto the adhesive on the second surface of the second release liner.

13. The method of forming a patch of claim 12, wherein the second adhesive is a hot melt adhesive, solvent acrylic adhesive, solvent acrylic-rubber hybrid adhesive, emulsion acrylic adhesive or contact adhesive.

14. The method of forming a patch of claim 12, wherein the second adhesive is the same as the first adhesive.

## Patentansprüche

1. Hautpflaster mit verlängerter Tragezeit, umfassend:
(a) ein Polyurethanvliesstoffobermaterial; und
(b) Hybridklebstoffmatrix, hergestellt mit Acrylpolymeren, die mit Kautschukpolymeren copolymerisiert sind, wobei die Hybridklebstoffmatrix eine Wasserdampfdurchlässigkeitsrate von weniger als 400 (g/m²)/Tag aufweist, wobei die Wasserdampfdurchlässigkeitsrate mit einem umgekehrten Becherverfahren mit einer Klebstoffbeschichtung auf 50 g/m² gemessen wird, wie in der Beschreibung beschrieben.

2. Pflaster nach Anspruch 1, wobei das Polyurethanvliesstoffsubstrat eine Dicke von 150 bis 250 µm und ein Gewicht von 50 bis 125 g/m² aufweist.

3. Pflaster nach Anspruch 1, wobei das Acrylpolymer mit einem Gemisch von Acrylatmonomeren hergestellt ist, ausgewählt aus der Gruppe bestehend aus Acrylsäure, Methacrylsäure, Beta-Carboxyethylacrylat, 2-Hydroxyethylacrylat, 2-Hydroxyethylmethacrylat, Hydroxypropylacrylat, Hydroxybutylacrylat, mit tertiärem Amin funktionalisierten Alkylacrylatmonomeren und Gemischen davon; und wobei das Kautschukpolymer hergestellt ist aus der Gruppe bestehend aus Isopren, Butadien, Isobutylen, hydriertem Isopren, hydriertem Butadien, hydriertem Isobutylen und Gemischen davon.

4. Pflaster nach Anspruch 3, wobei der Klebstoff ferner Weichmacher, Klebrigmacher, Haftvermittler, Initiator, Lösungsmittel, Antioxidationsmittel und Zusatzstoffe umfasst.

5. Pflaster nach Anspruch 1, ferner umfassend eine Trennlage, die ausgewählt ist aus der Gruppe bestehend aus silikonisiertem getrennten Papier oder silikonisierter getrennter PET-Folie.

6. Pflaster nach Anspruch 1, das ein medizinisches Pflaster ist.

7. Vorrichtung, umfassend:
a. ein Hautpflaster mit verlängerter Tragezeit, umfassend
i. eine Klebstoffmatrix, die eine Wasserdampfdurchlässigkeitsrate von weniger als 400 (g/m²)/Tag aufweist, wobei die Wasserdampfdurchlässigkeitsrate mit einem umgekehrten Becherverfahren mit einer Klebstoffbeschichtung auf 50 g/m² gemessen wird, wie in der Beschreibung beschrieben; und
ii. ein flexibles Polyurethanvliesstoffobermaterial;
b. einen zweiten Klebstoff; und
c. einen Detektor.

8. Vorrichtung nach Anspruch 7, wobei die Klebstoffmatrix ein Acryl-Kautschuk-Hybridklebstoff oder ein Lösungsmittelacrylklebstoff ist.

9. Vorrichtung nach Anspruch 8, wobei der Detektor zum Erfassen oder Messen eines physiologischen Zeichens geeignet ist, das ausgewählt ist aus der Gruppe bestehend aus einer elektrischen Veränderung, einer biochemischen Veränderung, einer Temperaturveränderung, einer Bewegungsveränderung, einer Aktivitätsveränderung, einer Haltungsveränderung und Mischungen davon.

10. Verfahren zum Ausbilden eines Hautpflasters mit verlängerter Tragezeit, umfassend:
a. Herstellen eines Polyurethanvliesstoffobermaterials, das eine erste Seite und eine zweite Seite aufweist;
b. Herstellen einer Trennlage, die eine erste Seite und eine zweite Seite aufweist, wobei auf die erste Seite eine Silikontrennbeschichtung aufgetragen wird;
c. Herstellen eines ersten Klebstoffs, der eine Wasserdampfdurchlässigkeitsrate von weniger als 400 (g/m²)/Tag aufweist, wobei die Wasserdampfdurchlässigkeitsrate mit einem umgekehrten Becherverfahren mit einer Klebstoffbeschichtung auf 50 g/m² gemessen wird, wie in der Beschreibung beschrieben;
d. Beschichten des ersten Klebstoffs auf die Trennbeschichtung auf der ersten Seite der Trennlage; und
e. Laminieren des ersten Klebstoffs auf die erste Seite des Polyurethanvliesstoffsubstrats;
wobei der Klebstoff zwischen der ersten Seite des Polyurethanvliesstoffobermaterials und der ersten Seite der Trennlage sandwichartig eingeschlossen ist.

11. Verfahren zum Ausbilden eines Pflasters nach Anspruch 10, wobei der erste Klebstoff mit einem Acryl-Kautschuk-Hybridpolymer, einem Weichmacher, einem Klebrigmacher, einem Haftvermittler, einem Initiator und einem Zusatzstoff hergestellt wird.

12. Verfahren zum Ausbilden eines Pflasters nach Anspruch 11, ferner umfassend die Schritte nach Schritt d,
e. Herstellen eines zweiten Klebstoffs;
f. Herstellen einer zweiten Trennlage, die eine erste Oberfläche und eine zweite Oberfläche aufweist;
g. Beschichten des zweiten Klebstoffs auf die zweite Oberfläche der zweiten Trennlage;
h. Herstellen eines Detektors; und
i. Auftragen des Detektors auf den Klebstoff auf die zweite Oberfläche der zweiten Trennlage.

13. Verfahren zum Ausbilden eines Pflasters nach Anspruch 12, wobei der zweite Klebstoff ein Schmelzklebstoff, ein Lösungsmittelacrylklebstoff, ein Lösungsmittelacrylkautschuk-Hybridklebstoff, ein Emulsionsacrylklebstoff oder ein Kontaktklebstoff ist.

14. Verfahren zum Ausbilden eines Pflasters nach Anspruch 12, wobei der zweite Klebstoff derselbe wie der erste Klebstoff ist.

## Revendications

1. Timbre cutané à port prolongé comprenant :
(a) une pellicule frontale non-tissée en polyuréthane ; et
(b) une matrice adhésive hybride préparée avec des polymères acryliques copolymérisés avec des polymères de caoutchouc, dans lequel la matrice adhésive hybride a un taux de perméabilité à la vapeur d'eau inférieur à 400 (g/m²)/jour, dans lequel le taux de perméabilité à la vapeur d'eau est mesuré par un procédé de coupelle inversée avec un revêtement détachable sur 50 g/m² comme décrit dans la description.

2. Timbre selon la revendication 1, dans lequel le substrat non-tissé en polyuréthane a une épaisseur de 150 à 250 µm et un poids de 50 à 125 g/m².

3. Timbre selon la revendication 1, dans lequel le polymère acrylique est préparé avec un mélange de monomères acryliques choisis dans le groupe constitué d'acide acrylique, acide méthacrylique, acrylate de bêta-carboxyéthyle, acrylate de 2-hydroxyéthyle, méthacrylate de 2-hydroxyéthyle, acrylate d'hydroxypropyle, acrylate d'hydroxybutyle, des monomères d'acrylate d'alkyle fonctionnalisés par amine tertiaire, et leurs mélanges ; et dans lequel le polymère de caoutchouc est préparé à partir du groupe constitué d'isoprène, butadiène, isobutylène, isoprène hydrogéné, butadiène hydrogéné, isobutylène hydrogéné et leurs mélanges.

4. Timbre selon la revendication 3, dans lequel l'adhésif comprend en outre un plastifiant, un agent collant, un promoteur d'adhésion, un initiateur, un solvant, un antioxydant et un additif.

5. Timbre selon la revendication 1, comprenant en outre une protection détachable choisie dans le groupe constitué de papier antiadhésif siliconé ou de film PET antiadhésif siliconé.

6. Timbre selon la revendication 1, qui est un timbre médical.

7. Dispositif comprenant :
a. un timbre cutané à port prolongé comprenant
i. une matrice adhésive ayant un taux de perméabilité à la vapeur d'eau inférieur à 400 (g/m²)/jour, dans lequel le taux de perméabilité à la vapeur d'eau est mesuré à l'aide d'un procédé de coupelle inversée avec un revêtement détachable de 50 g/m² comme décrit dans la description ; et
ii. une pellicule frontale non-tissée en polyuréthane souple ;
b. un second adhésif ; et
c. un détecteur.

8. Dispositif selon la revendication 7, dans lequel la matrice adhésive est un adhésif hybride acrylique-caoutchouc ou un adhésif acrylique à base de solvant.

9. Dispositif selon la revendication 8, dans lequel le détecteur est adapté à la détection ou à la mesure d'un signe physiologique choisi dans le groupe constitué d'un changement électrique, d'un changement biochimique, d'un changement de température, d'un changement de mouvement, d'un changement d'activité, d'un changement de posture, et de leurs mélanges.

10. Procédé de formation d'un timbre cutané à port prolongé comprenant :
a. la préparation d'une pellicule frontale non-tissée en polyuréthane ayant une première face et une seconde face ;
b. la préparation d'une protection détachable ayant une première face et une seconde face, dans lequel un revêtement détachable en silicone est appliqué sur la première face ;
c. la préparation d'un premier adhésif ayant un taux de perméabilité à la vapeur d'eau inférieur à 400 (g/m²)/jour, dans lequel le taux de perméabilité à la vapeur d'eau est mesuré à l'aide d'un procédé de coupelle inversée avec un revêtement détachable de 50 g/m² comme décrit dans la description ;
d. le revêtement du premier adhésif sur le revêtement détachable sur la première face de la protection détachable ; et
e. la stratification du premier adhésif sur la première face du substrat non tissé en polyuréthane ;
moyennant quoi l'adhésif est pris en sandwich entre la première face de la pellicule frontale non-tissée en polyuréthane et la première face de la protection détachable.

11. Procédé de formation d'un timbre selon la revendication 10, dans lequel le premier adhésif est préparé avec un polymère hybride acrylique-caoutchouc, un plastifiant, un agent adhésif, un promoteur d'adhésion, un initiateur et un additif.

12. Procédé de formation d'un timbre selon la revendication 11, comprenant en outre les étapes après l'étape d consistant à,
e. préparer un second adhésif ;
f. préparer une seconde protection détachable, ayant une première surface et une seconde surface ;
g. revêtir le second adhésif sur la seconde surface de la seconde protection détachable ;
h. préparer un détecteur ; et
i. appliquer le détecteur sur l'adhésif sur la seconde surface de la seconde protection détachable.

13. Procédé de formation d'un timbre selon la revendication 12, dans lequel le second adhésif est un adhésif thermofusible, un adhésif acrylique à solvant, un adhésif hybride acrylique-caoutchouc à solvant, un adhésif acrylique à émulsion ou un adhésif de contact.

14. Procédé de formation d'un timbre selon la revendication 12, dans lequel le second adhésif est le même que le premier adhésif.
